# EUROPEAN PATENT SPECIFICATION

(11) **EP 2 814 398 B1**
(45) Date of publication and mention of the grant of the patent: **28.06.2017**
(21) Application number: 13716836.5
(22) Date of filing: 11.02.2013
(51) Int. Cl.: A61B 8/08, A61B 8/00, G01S 7/52, G01S 15/89, G06T 15/08

(54) **SIMULTANEOUS ULTRASONIC VIEWING OF 3D VOLUME FROM MULTIPLE DIRECTIONS**
GLEICHZEITIGE ULTRASCHALLBETRACHTUNG VON 3D-VOLUMEN AUS MEHREREN RICHTUNGEN
VISUALISATION ULTRASONORE SIMULTANÉE DE VOLUME EN 3D DANS DE MULTIPLES DIRECTIONS

(30) Priority: 13.02.2012 US 201261597931 P
(43) Date of publication of application: 24.12.2014
(73) Proprietor: Koninklijke Philips N.V., 5656 AE Eindhoven (NL)
(72) Inventor: PRATER, David, NL-5656 AE Eindhoven (NL); WATKINS, Stephen, P., NL-5656 AE Eindhoven (NL); ADAMS, David Frank, NL-5656 AE Eindhoven (NL)
(74) Representative: Steffen, Thomas
(86) International application number: PCT/IB2013/051118
(87) International publication number: WO 2013/121341

(56) References cited:
- EP-A1- 2 182 382
- US-B1- 6 245 017
- FENSTER A ET AL: "THREE-DIMENSIONAL ULTRASOUND IMAGING", PHYSICS IN MEDICINE AND BIOLOGY, INSTITUTE OF PHYSICS PUBLISHING, BRISTOL GB, vol. 46, no. 5, 1 May 2001 (2001-05-01), XP001205497, ISSN: 0031-9155, DOI: 10.1088/0031-9155/46/5/201

## Description

This invention relates to medical diagnostic ultrasound systems and, in particular, to ultrasonic imaging systems which display a 3D volume in simultaneous views from multiple directions.

Ultrasonic diagnostic imaging system have traditionally been used to image a plane of the body in real time. A probe with a one dimensional (1D) array transducer or mechanically swept single element transducer can be operated to repeatedly scan a plane of the body to produce real time image sequences for live display of the anatomy. Recently two dimensional (2D) array transducers and mechanically swept 1D arrays have been developed for scanning a volumetric region of the body. Such probes can be used to produce three dimensional (3D) images of the volume being scanning, also in real time. A display technique commonly used for 3D display of ultrasonically scanned volumes is called kinetic parallax, in which a 3D data set of the volume is rendered from a series of different viewing directions. As the operator moves a control on the ultrasound system to change the viewing direction, the volume rendering processor renders the volume in a newly selected viewing direction and the progression of different directions gives the appearance of a 3D volume moving on the display screen. Individual planes can be selected from a three dimensional data set for viewing, a technique known as multiplanar reconstruction (MPR). A system according to the preamble of claim 1 is known from EP 2 182 382 A1.

It is at times desirable to view a volumetric region of interest (ROI) from different directions. With a conventional viewer this must be done by viewing the ROI from one direction, then turning or rotating the 3D ROI so that it can be seen from the second direction. A comparison of the two views must be done by remembering what was seen in the first view, then moving the view to the second direction and making the comparison based on the recollection of the first view. For comparison of subtle anatomical differences, it would be preferable not to rely on memorization, or moving the views back and forth to try to make the diagnosis. It would be preferable to be able to see both views simultaneously so that the clinician is seeing both views at the same time while making the diagnosis.

In accordance with the principles of the present invention, a diagnostic ultrasound system is described which enables a clinician to view a volume from multiple external viewing perspectives at the same time. When the clinician manipulates one view, the manipulation is applied to the second view so that both views are changed in unison, as the clinician would expect the views to change if both were altered in the same way. Either or both views can also be interrogated by MPR viewing. A system of the present invention is particularly useful for guiding an invasive device such as a needle or a catheter inside the body.

In the drawings:
FIGURE 1 illustrates in block diagram form an ultrasonic diagnostic imaging system constructed in accordance with the principles of the present invention.
FIGURE 2 shows a cubic ROI and two different viewing orientations.
FIGURES 3a-3d illustrate simultaneous changes of two viewing orientations of the cubic ROI of FIGURE 2 by manipulation of one of the views.
FIGURE 4 illustrates two simultaneous views of the cubic ROI of FIGURE 2 from orthogonal viewing orientations.
FIGURES 5a-5c illustrate simultaneous views from different directions of a volumetric ROI including a heart valve.
FIGURES 6a-6c illustrate simultaneous views of a catheter procedure from orthogonal viewing directions.

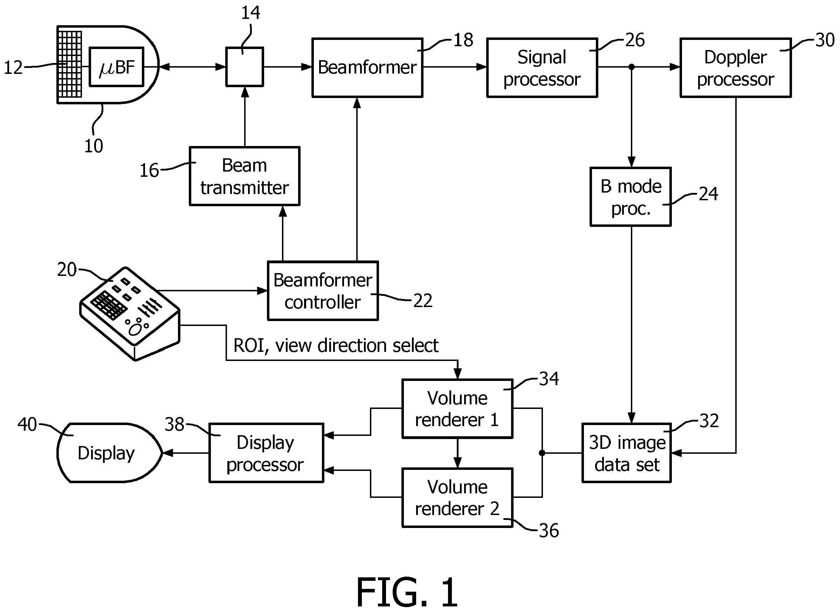

Referring first to FIGURE 1, an ultrasonic diagnostic imaging system constructed in accordance with the principles of the present invention is shown in block diagram form. An ultrasound probe 10 capable of three dimensional imaging includes a two dimensional array transducer 12 which transmits electronically steered and focused beams over a volumetric region and receives single or multiple receive beams in response to each transmit beam. Groups of adjacent transducer elements referred to as "patches" or "subarrays" are integrally operated by a microbeamformer (µBF) in the probe 12, which performs partial beamforming of received echo signals and thereby reduces the number of conductors in the cable between the probe and the main system. Suitable two dimensional arrays are described in U.S. Patent 6,419,633 (Robinson et al.) and in U.S. Patent 6,368,281 (Solomon et al.) Microbeamformers are described in U.S. Patents 5,997,479 (Savord et al.) and 6,013,032 (Savord). The transmit beam characteristics of the array are controlled by a beam transmitter 16, which causes the apodized aperture elements of the array to emit a focused beam of the desired breadth in a desired direction through a volumetric region of the body. Transmit pulses are coupled from the beam transmitter 16 to the elements of the array by means of a transmit/receive switch 14. The echo signals received by the array elements and microbeamformer in response to a transmit beam are coupled to a system beamformer 18, where the partially beamformed echo signals from the microbeamformer are processed to form fully beamformed single or multiple receive beams in response to a transmit beam. A suitable beamformer for this purpose is described in the aforementioned Savord '032 patent.

The receive beams formed by the beamformer 18 are coupled to a signal processor 26 which performs functions such as filtering and quadrature demodulation. The echo signals of the processed receive beams are coupled to a Doppler processor 30 and/or a B mode processor 24. The Doppler processor 30 processes the echo information into Doppler power or velocity information signals. For B mode imaging the receive beam echoes are envelope detected and the signals logarithmically compressed to a suitable dynamic range by the B mode processor 24. The echo and Doppler signals from the scanned volumetric region are processed to form one or more 3D image datasets which are stored in a 3D image dataset buffer 32. The 3D image data may be processed for display in several ways. One way is to produce multiple 2D planes of the volume. This is described in U.S. patent 6,443,896 (Detmer). Such planar images of a volumetric region are produced by a multi-planar reformatting as is known in the art. In accordance with the present invention, the three dimensional image data may also be rendered to form perspective or kinetic parallax 3D displays by volume renderers 34 and 36. The resulting images, which may be B mode, Doppler or both as described in US patent 5,720,291 (Schwartz), are coupled to a display processor 38, from which they are displayed on an image display 40. User control of the beamformer controller 22, the selection of an ROI, the selection of directions in which the ROI is to be viewed, and other functions of the ultrasound system are provided through a user interface or control panel 20.

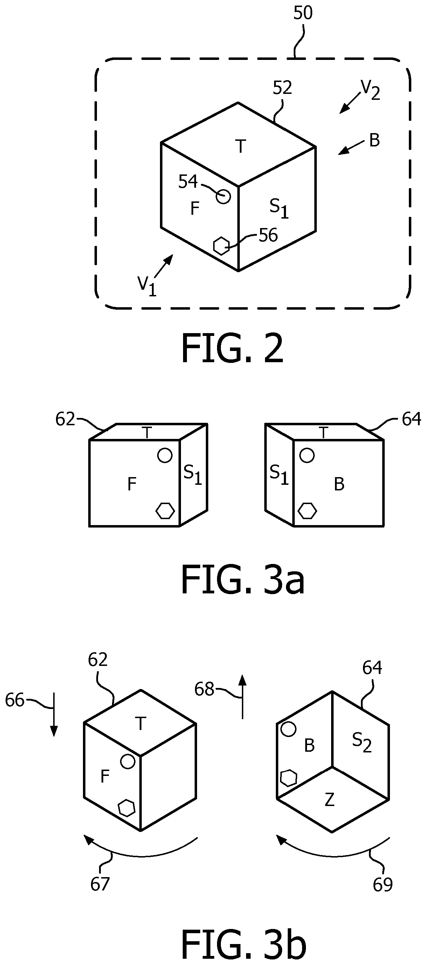

A clear understanding of manipulation of simultaneous views of a 3D ROI may be had with reference to FIGURES 2-4. In these drawings a cubic ROI 52 located in a volumetric region 50 is used for clarity of illustration. As seen in FIGURE 2, the cubic ROI 52 has a front face F, a top face T, side faces S₁ and S₂, and back (B) and bottom (Z) faces, the latter three not visible in FIGURE 2. The 3D ROI 52 has two passageways extending from the front face to the back face, one drawn as a circular passageway 54 and the other drawn as a hexagonal passageway 56. Two viewing directions V₁ and V₂ are also shown in FIGURE 2, which view the 3D ROI from the front F and the back B, respectively.

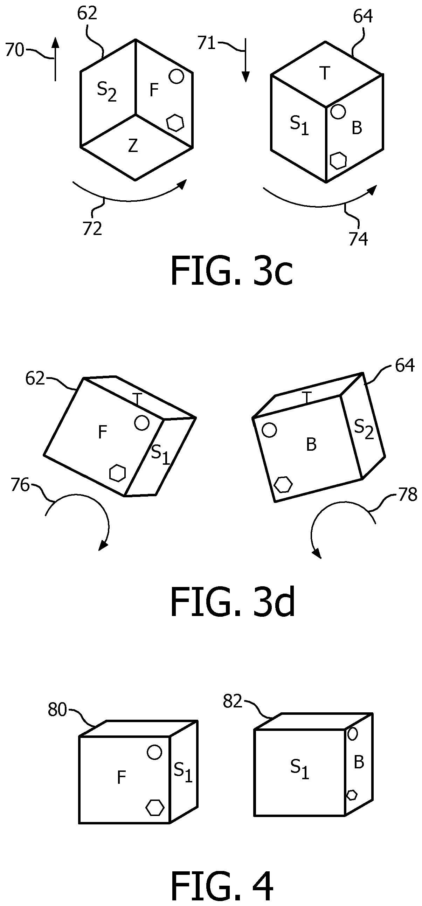

FIGURES 3a-4 show simultaneous 3D views of the 3D ROI formed by simultaneous operation of volume renderer1 and volume renderer2 in accordance with the principles of the present invention. The two 3D views are displayed to the clinician simultaneously on the display 40 as illustrated in these drawings. Volume renderer1 renders the 3D ROI as viewed looking toward the front face F and volume renderer2 renders the 3D ROI as viewed looking toward the back face B. The viewing directions used for rendering are thus opposed to each other by 180°. In the front face view 62 of FIGURE 3a the viewing direction is slightly to the right of and above the front face of the 3D ROI so that the top T and side S₁ faces can be seen. For the back face view 64 the viewing direction is slightly to the left of and above the back face B so that the side S₁ and top T faces can also be seen in this view. Slight variation from exactly 180° views can be used as shown in FIGURE 3a, or both views can be exactly 180° in opposition as shown in FIGURE 4. As FIGURE 3a illustrates, the passageways 54,56 extending through the 3D ROI are seen on the right side of the front face F and on the left side of the back face B as a clinician would expect to see them.

In FIGURE 3b the clinician has manipulated a control of the user interface such as a trackball on the control panel 20 or a softkey control on the display screen to rotate the 3D ROI 62 on the left side of the display slightly to the left as indicated by arrow 67. The clinician has also manipulated a user control to tilt the 3D ROI slightly downward as indicated by arrow 66 so that more of the top face T can be seen. As the clinician manipulates the left 3D ROI 62 in this way, the 3D ROI view 64 on the right moves in correspondence, as if the clinician manipulated the right view to move in the same way. The right view 64 from the back of the 3D ROI rotates the same amount to the left as indicated by the arrow 69 and tilts upward by the same amount (arrow 68) as the tilt of the left 3D ROI view, causing more of the bottom face Z to be visible. Thus, by manipulating one view of the 3D ROI, the corresponding adjustments are made to the other view of the 3D ROI. The clinician has the sense of moving one 3D ROI with the control adjustments and seeing the resulting change in both views of the front and back of the 3D ROI as if clinician were seeing the same ROI and its motion from two different views.

FIGURE 3c illustrates the front and back 3D ROI views 62 and 64 after the clinician has rotated the ROI to the right (as indicated by arrows 72 and 74) and tilted the front view of the ROI up (as indicated by arrows 70) so that the bottom face Z is visible. As the drawing indicates, the back view 64 moves in a corresponding manner. The upward tilt 70 of the ROI as seen from the front is seen as a downward tilt from the back as indicated by arrow 71, causing the top face T to be more visible from the back. Both the left and right views move in unison as the clinician adjusts the orientation of one of the views.

FIGURE 3d illustrates the result of rotating the left view to tilt the right side of the 3D ROI 62 downward. As this happens, the rear view 64 of the 3D ROI tilts down on the left side as indicated by arrow 78. This is how the clinician would expect the right view to behave when rotating the left view: the S₁ face side tilts down in both views. The same result can be obtained by tilting the right view 64 downward on the left side, which causes the corresponding effect of tilting the right side of view 62 down to the right. Thus, moving the ROI in one of the views causes the same movement of the other view, which is seen from the different viewing orientation.

FIGURE 4 shows two views of a 3D ROI, with the left view 80 looking at the 3D ROI from the front face F and the right view 82 looking at the 3D ROI from the side face S₁. As in the previous examples, manipulating one of the views of the 3D ROI will cause the same motion of the 3D ROI in the other view but as seen from a different viewpoint. The two views of the 3D ROI can thus be at a 180° angle to each other as shown in FIGURES 3a-3d, or at a 90° angle to each other as shown in FIGURE 4, or at any other intermediate angle between the views, *e.g.,* between 0° and 180°.

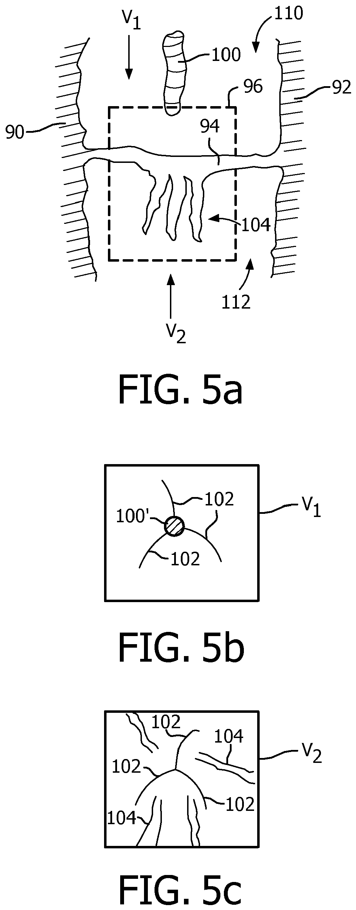

FIGURES 5a-5c illustrate a clinical application of an ultrasound system of the present invention. In this example a catheter 100 has been threaded into an atrium 110 of a heart in preparation for passage through a mitral or tricuspid valve 94 and into a ventricle 112. The heart valve 94 is seen to be attached to the myocardial walls 90 and 92 on opposite sides of the heart. Extending from the valve leaflets in the ventricle are chordate tendineae 104, cord-like tendons that attach the valve leaflets to papillary muscles in the ventricle. An ultrasound system of the present invention is used to guide the catheter procedure by imaging the heart as illustrated in FIGURE 5a and defining within such a volumetric region a 3D ROI 96. As FIGURE 5a illustrates, this 3D ROI extends into the heart chambers on both sides of the valve and includes the valve through which the catheter 100 is to be inserted. With the 3D ROI defined in this way, the 3D ROI is viewed simultaneously from both the face in the atrium 110 and the face in the ventricle as shown in FIGURES 5b and 5c. In the view V₁ from the atrium 110 as shown in FIGURE 5b, the clinician can see the catheter 100' as it approaches the slits 102 between the valve leaflets. On the other side of the valve the V2 view of FIGURE 5c views the slits 102 of the valve leaflets through which the catheter will soon appear, and the chordate tendineae 104 extending back from the valve leaflets. By viewing the valve 94 from both sides in 3D, the clinician can guide the catheter 100 toward the center of the heart valve 94, and view its insertion through the heart valve as the catheter appears on the ventricular side of the valve 94.

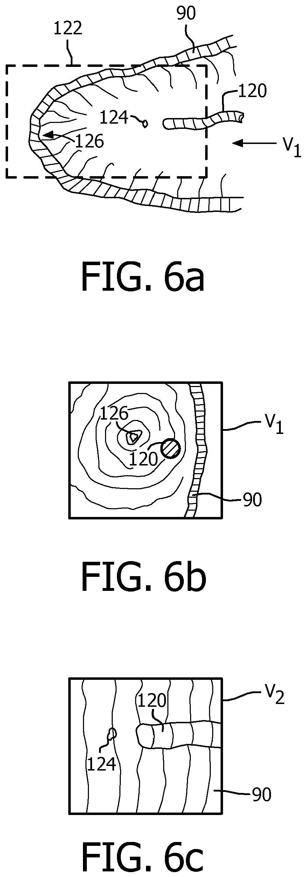

FIGURES 6a-6c illustrate another example of a clinical procedure performed with an ultrasound system of the present invention. In this example the 3D ROI is viewed in two orthogonal viewing directions V₁ and V₂. In this example a catheter 120 is being guided to perform a clinical procedure on a spot 124 on the wall of the myocardium 90 of a heart. A 3D ROI is delineated as shown by outline 122 in FIGURE 6a, which includes the catheter 120, the spot 124 which is to be treated, and the far side 126 of the heart chamber in which the procedure is to be performed. This 3D ROI 122 is viewed in two orthogonal viewing directions, V₁ as shown in FIGURE 6a, and in a second direction looking into the plane of the FIGURE 6a drawing. FIGURE 6b illustrates the 3D ROI 122 as viewed from direction V₁. In this view the catheter 120 can be axially seen alongside the wall 90 of the myocardium and approaching the far end 126 of the heart chamber in which the catheter is located. The orthogonal V2 view is shown in FIGURE 6c. In this view the catheter 120 is seen approaching point 124 at which the procedure is to be performed and is in an orientation approximately parallel to the heart wall 90. The two orthogonal views give the clinician a sense of how the catheter is proceeding along the heart wall, its spacing from the heart wall, and how much further the catheter needs to be extended to reach the point 124 at which the procedure is to be performed.

## Claims

1. An ultrasonic diagnostic imaging system comprising:
an ultrasound probe (10) operable to scan a volumetric region of a body which produces echo signals from three dimensions of the region (50);
a signal processor (26), responsive to the echo signals from the volumetric region, which produces a 3D image data set (32) of the region;
a first user control which provides a selection of a first and a second viewing direction, the second viewing direction being different from the first viewing direction;
a volume renderer coupled to receive the 3D image data set and arranged to produce a first and a second 3D view of the region corresponding to the first and the second viewing direction; and
a display, responsive to the volume renderer, which is arranged to display the first and the second 3D view **characterized in that**
the volume renderer comprises a first volume renderer (34) arranged to produce the first 3D view and a second volume renderer (36) arranged to produce the second 3D view, wherein the second volume renderer is adapted to operate simultaneously with the first volume renderer; and
wherein the display is arranged to display the first and the second 3D view simultaneously.

2. The ultrasonic diagnostic imaging system of Claim 1, wherein the two different viewing directions further comprise views of the region as seen from directions oriented 180° with respect to each other.

3. The ultrasonic diagnostic imaging system of Claim 1, wherein the two different viewing directions further comprise views of the region as seen from directions oriented 90° with respect to each other.

4. The ultrasonic diagnostic imaging system of Claim 1, wherein the two different viewing directions further comprise views of the region as seen from directions oriented at an angle between 0° and 180° with respect to each other.

5. The ultrasonic diagnostic imaging system of Claim 1, further comprising a second user control operable by a user to select a 3D region of interest (ROI) within the volumetric region.

6. The ultrasonic diagnostic imaging system of Claim 5, further comprising an invasive object which can be seen on the display when manipulated in the volumetric region;
wherein the region of interest further contains anatomy of interest,
wherein the system is configured to visualize the invasive object moving away from a viewer in the first 3D view when manipulated in a first direction in relation to the anatomy of interest, and
wherein the system is configured to simultaneously visualize the invasive object moving toward the the viewer in the second 3D view when manipulated in the first direction in relation to the anatomy of interest.

7. The ultrasonic diagnostic imaging system of Claim 5, further comprising an invasive object which can be seen on the display when manipulated in the volumetric region;
wherein the region of interest further contains anatomy of interest,
wherein the system is configured to visualize the invasive object moving toward or away from a viewer in the first 3D view when manipulated in a first direction in relation to the anatomy of interest, and
wherein the system is further configured to simultaneously visualize the invasive object moving laterally with respect to the viewer in the second 3D view when manipulated in the first direction in relation to the anatomy of interest.

8. The ultrasonic diagnostic imaging system of Claim 1, further comprising a third user control, coupled to the volume renderers, which is operable to change the orientation of the volumetric region as seen from the two different viewing directions.

9. The ultrasonic diagnostic imaging system of Claim 8, wherein the third user control is operable to change the orientation of the volumetric region as seen from the first viewing direction,
wherein the orientation of the volumetric region as seen from the second viewing direction is changed in correspondence to the change applied to the first viewing direction,
wherein a user sees a single change in the orientation of the volumetric region as it would appear from two different viewing directions of the volumetric region.

10. The ultrasonic diagnostic imaging system of Claim 9, wherein the system is configured such that the change in the orientation of the two 3D views of the volumetric region produced by manipulation of the third user control is visualized in real time.

11. The ultrasonic diagnostic imaging system of Claim 8, wherein the third user control is further operable to tilt the two 3D views up or down, turn the two 3D views left or right, or rotated the two 3D views clockwise or counter-clockwise.

12. The ultrasonic diagnostic imaging system of Claim 11 the system is configured such that:
when the third user control is operated to tilt one of the 3D views up, the other 3D view tilts down correspondingly;
when the third user control is operated to turn one of the 3D views to the left, the other 3D view turns to the left correspondingly; and
when the third user control is operated to rotate one of the two 3D views clockwise, the other 3D view rotates counter-clockwise correspondingly.

13. The ultrasonic diagnostic imaging system of Claim 1, wherein the 3D image data set further comprises B mode or Doppler image data.

## Patentansprüche

1. Ultraschallsystem zur diagnostischen Bildgebung, umfassend:
eine Ultraschallsonde (10), die betriebsfähig ist, um eine volumetrische Region eines Körpers abzutasten, die Echosignale aus drei Dimensionen der Region (50) erzeugt;
einen Signalprozessor (26), der auf die Echosignale aus der volumetrischen Region reagiert und einen 3D-Bilddatensatz (32) der Region erzeugt;
eine erste Benutzersteuerung, die eine Auswahl von einer ersten und einer zweiten Blickrichtung bereitstellt, wobei sich die zweite Blickrichtung von der ersten Blickrichtung unterscheidet;
eine Volumenrendering-Einheit, die gekoppelt ist, um den 3D-Bilddatensatz zu empfangen, und vorgesehen ist, um eine erste und eine zweite 3D-Ansicht der Region zu erzeugen, die der ersten und der zweiten Blickrichtung entsprechen; und
eine Anzeige, die auf die Volumenrendering-Einheit reagiert und vorgesehen ist, um die erste und die zweite Ansicht anzuzeigen,
**dadurch gekennzeichnet, dass**
die Volumenrendering-Einheit eine erste Volumenrendering-Einheit (34) zum Erzeugen der ersten 3D-Ansicht und eine zweite Volumenrendering-Einheit (36) zum Erzeugen der zweiten 3D-Ansicht umfasst, wobei die zweite Volumenrendering-Einheit dafür ausgelegt ist, gleichzeitig mit der ersten Volumenrendering-Einheit zu arbeiten; und
wobei die Anzeige dafür vorgesehen ist, die erste und die zweite 3D-Ansicht gleichzeitig anzuzeigen.

2. Ultraschallsystem zur diagnostischen Bildgebung nach Anspruch 1, wobei die beiden verschiedenen Blickrichtungen weiterhin Ansichten der Region gesehen aus Richtungen umfassen, die 180° in Bezug zueinander ausgerichtet sind.

3. Ultraschallsystem zur diagnostischen Bildgebung nach Anspruch 1, wobei die beiden verschiedenen Blickrichtungen weiterhin Ansichten der Region gesehen aus Richtungen umfassen, die 90° in Bezug zueinander ausgerichtet sind.

4. Ultraschallsystem zur diagnostischen Bildgebung nach Anspruch 1, wobei die beiden verschiedenen Blickrichtungen weiterhin Ansichten der Region gesehen aus Richtungen umfassen, die in einem Winkel zwischen 0° und 180° in Bezug zueinander ausgerichtet sind.

5. Ultraschallsystem zur diagnostischen Bildgebung nach Anspruch 1, weiterhin umfassend eine zweite Benutzersteuerung, die durch einen Benutzer betätigt werden kann, um eine interessierende 3D-Region (ROI) innerhalb der volumetrischen Region auszuwählen.

6. Ultraschallsystem zur diagnostischen Bildgebung nach Anspruch 5, weiterhin umfassend ein invasives Objekt, das auf der Anzeige zu sehen ist, wenn es in der volumetrischen Region manipuliert wird;
wobei die interessierende Region weiterhin interessierende Anatomie enthält,
wobei das System konfiguriert ist, um das invasive Objekt, während es sich von einem Betrachter weg bewegt, in der ersten 3D-Ansicht zu visualisieren, wenn es in einer ersten Richtung in Bezug auf die interessierende Anatomie manipuliert wird, und
wobei das System konfiguriert ist, um gleichzeitig das invasive Objekt, während es sich auf den Betrachter zu bewegt, in der zweiten 3D-Ansicht zu visualisieren, wenn es in einer ersten Richtung in Bezug auf die interessierende Anatomie manipuliert wird.

7. Ultraschallsystem zur diagnostischen Bildgebung nach Anspruch 5, weiterhin umfassend ein invasives Objekt, das auf der Anzeige zu sehen ist, wenn es in der volumetrischen Region manipuliert wird;
wobei die interessierende Region weiterhin interessierende Anatomie enthält,
wobei das System konfiguriert ist, um das invasive Objekt, während es sich von einem Betrachter weg oder auf diesen zu bewegt, in der ersten 3D-Ansicht zu visualisieren, wenn es in einer ersten Richtung in Bezug auf die interessierende Anatomie manipuliert wird, und
wobei das System weiterhin konfiguriert ist, um gleichzeitig das invasive Objekt, während es sich seitlich in Bezug auf den Betrachter bewegt, in der zweiten 3D-Ansicht zu visualisieren, wenn es in der ersten Richtung in Bezug auf die interessierende Anatomie manipuliert wird.

8. Ultraschallsystem zur diagnostischen Bildgebung nach Anspruch 1, weiterhin umfassend eine dritte Benutzersteuerung, die mit den Volumenrendering-Einheiten gekoppelt ist und betriebsfähig ist, um die Ausrichtung der volumetrischen Region, gesehen aus den beiden verschiedenen Blickrichtungen, zu ändern.

9. Ultraschallsystem zur diagnostischen Bildgebung nach Anspruch 8, wobei die dritte Benutzersteuerung betriebsfähig ist, um die Ausrichtung der volumetrischen Region, gesehen aus der ersten Blickrichtung, zu ändern,
wobei die Ausrichtung der volumetrischen Region, gesehen aus der zweiten Blickrichtung, entsprechend der Änderung geändert wird, die auf die erste Blickrichtung angewandt wird,
wobei ein Benutzer eine einzelne Änderung in der Ausrichtung der volumetrischen Region sieht, wie sie aus zwei verschiedenen Blickrichtungen der volumetrischen Region erscheinen würde.

10. Ultraschallsystem zur diagnostischen Bildgebung nach Anspruch 9, wobei das System derartig konfiguriert ist, dass die Änderung in der Ausrichtung der beiden 3D-Ansichten der volumetrischen Region, die durch Manipulation der dritten Benutzersteuerung erzeugt wurde, in Echtzeit visualisiert wird.

11. Ultraschallsystem zur diagnostischen Bildgebung nach Anspruch 8, wobei die dritte Benutzersteuerung weiterhin betriebsfähig ist, um die beiden 3D-Ansichten nach oben oder unten zu neigen, die beiden 3D-Ansichten nach links oder rechts zu wenden oder die beiden 3D-Ansichten mit dem Uhrzeigersinn oder gegen den Uhrzeigersinn zu drehen.

12. Ultraschallsystem zur diagnostischen Bildgebung nach Anspruch 11, wobei das System derartig konfiguriert ist, dass:
wenn die dritte Benutzersteuerung betätigt wird, um eine der 3D-Ansichten nach oben zu neigen, die andere 3D-Ansicht entsprechend nach unten geneigt wird;
wenn die dritte Benutzersteuerung betätigt wird, um eine der 3D-Ansichten nach links zu wenden, die andere 3D-Ansicht entsprechend nach links gewendet wird; und
wenn die dritte Benutzersteuerung betätigt wird, um eine der beiden 3D-Ansichten im Uhrzeigersinn zu drehen, die andere 3D-Ansicht entsprechend gegen den Uhrzeigersinn gedreht wird.

13. Ultraschallsystem zur diagnostischen Bildgebung nach Anspruch 1, wobei der 3D-Bilddatensatz weiterhin B-Mode- oder Doppler-Bilddaten umfasst.

## Revendications

1. Système d'imagerie diagnostique ultrasonore comprenant :
une sonde ultrasonore (10) permettant de balayer une région volumétrique d'un corps qui produit des signaux d'écho à partir de trois dimensions de la région (50) ;
un processeur de signaux (26), répondant aux signaux d'écho provenant de la région volumétrique, qui produit un ensemble de données d'images 3D (32) de la région ;
une première commande d'utilisateur qui fournit une sélection d'une première et d'une seconde direction de visualisation, la seconde direction de visualisation étant différente de la première direction de visualisation ;
un moteur de rendu de volume couplé pour recevoir l'ensemble de données d'images 3D et agencé pour produire une première et une seconde vue 3D de la région correspondant à la première et la seconde direction de visualisation ; et
un afficheur, répondant au moteur de rendu de volume, qui est agencé pour afficher la première et la seconde vue 3D
**caractérisé en ce que**
le moteur de rendu de volume comprend un premier moteur de rendu de volume (34) agencé pour produire la première vue 3D et un second moteur de rendu de volume (36) agencé pour produire la seconde vue 3D, dans lequel le second moteur de rendu de volume est adapté pour fonctionner simultanément avec le premier moteur de rendu de volume ; et
dans lequel l'afficheur est agencé pour afficher la première et la seconde vue 3D simultanément.

2. Système d'imagerie diagnostique ultrasonore de la revendication 1, dans lequel les deux directions de visualisation différentes comprennent en outre des vues de la région vue depuis des directions orientées à 180° l'une par rapport à l'autre.

3. Système d'imagerie diagnostique ultrasonore de la revendication 1, dans lequel les deux directions de visualisation différentes comprennent en outre des vues de la région vue depuis des directions orientées à 90° l'une par rapport à l'autre.

4. Système d'imagerie diagnostique ultrasonore de la revendication 1, dans lequel les deux directions de visualisation différentes comprennent en outre des vues de la région vue depuis des directions orientées à un angle entre 0° et 180° l'une par rapport à l'autre.

5. Système d'imagerie diagnostique ultrasonore de la revendication 1, comprenant en outre une deuxième commande d'utilisateur permettant à un utilisateur de sélectionner une région d'intérêt (ROI) 3D dans la région volumétrique.

6. Système d'imagerie diagnostique ultrasonore de la revendication 5, comprenant en outre un objet effractif qui peut être vu sur l'afficheur lorsqu'il est manipulé dans la région volumétrique ;
dans lequel la région d'intérêt contient en outre une anatomie d'intérêt,
dans lequel le système est configuré pour représenter l'objet effractif se déplaçant à l'opposé d'un observateur dans la première vue 3D lorsqu'il est manipulé dans une première direction en référence à l'anatomie d'intérêt, et
dans lequel le système est configuré pour représenter simultanément l'objet effractif se déplaçant vers l'observateur dans la seconde vue 3D lorsqu'il est manipulé dans la première direction en référence à l'anatomie d'intérêt.

7. Système d'imagerie diagnostique ultrasonore de la revendication 5, comprenant en outre un objet effractif qui peut être vu sur l'afficheur lorsqu'il est manipulé dans la région volumétrique ;
dans lequel la région d'intérêt contient en outre une anatomie d'intérêt,
dans lequel le système est configuré pour représenter l'objet effractif se déplaçant vers ou à l'opposé d'un observateur dans la première vue 3D lorsqu'il est manipulé dans une première direction en référence à l'anatomie d'intérêt, et
dans lequel le système est en outre configuré pour représenter simultanément l'objet effractif se déplaçant latéralement par rapport à l'observateur dans la seconde vue 3D lorsqu'il est manipulé dans la première direction en référence à l'anatomie d'intérêt.

8. Système d'imagerie diagnostique ultrasonore de la revendication 1, comprenant en outre une troisième commande d'utilisateur, couplée aux moteurs de rendu de volume, qui permet de changer l'orientation de la région volumétrique vue depuis les deux directions de visualisation différentes.

9. Système d'imagerie diagnostique ultrasonore de la revendication 8, dans lequel la troisième commande d'utilisateur permet de changer l'orientation de la région volumétrique vue depuis la première direction de visualisation,
dans lequel l'orientation de la région volumétrique vue depuis la seconde direction de visualisation est changée conformément au changement appliqué à la première direction de visualisation,
dans lequel un utilisateur voit un seul changement de l'orientation de la région volumétrique comme il apparaîtrait depuis deux directions de visualisation différentes de la région volumétrique.

10. Système d'imagerie diagnostique ultrasonore de la revendication 9, dans lequel le système est configuré de manière à ce que le changement de l'orientation des deux vues 3D de la région volumétrique produit par manipulation de la troisième commande d'utilisateur soit représenté en temps réel.

11. Système d'imagerie diagnostique ultrasonore de la revendication 8, dans lequel la troisième commande d'utilisateur permet en outre d'incliner les deux vues 3D vers le haut ou le bas, tourner les deux vues 3D vers la gauche ou la droite, ou tourner les deux vues 3D dans le sens des aiguilles d'une montre ou dans le sens inverse des aiguilles d'une montre.

12. Système d'imagerie diagnostique ultrasonore de la revendication 11, le système est configuré de manière à ce que :
lorsque la troisième commande d'utilisateur est actionnée pour incliner l'une des vues 3D vers le haut, l'autre vue 3D s'incline vers le bas de façon correspondante ;
lorsque la troisième commande d'utilisateur est actionnée pour tourner l'une des vues 3D vers la gauche, l'autre vue 3D tourne vers la gauche de façon correspondante ; et
lorsque la troisième commande d'utilisateur est actionnée pour faire tourner l'une des deux vues 3D dans le sens des aiguilles d'une montre, l'autre vue 3D tourne dans le sens inverse des aiguilles d'une montre de façon correspondante.

13. Système d'imagerie diagnostique ultrasonore de la revendication 1, dans lequel l'ensemble de données d'images 3D comprend en outre des données d'images de mode B ou Doppler.
